# EUROPEAN PATENT APPLICATION

(11) **EP 1 832 585 A1**
(43) Date of publication of application: **12.09.2007**
(21) Application number: 06110948.4
(22) Date of filing: 10.03.2006
(51) Int. Cl.: C07D 417/04, A61K 31/426

(54) **Thiazole-piperidine derivatives for treatment of hyperproliferative diseases**

(71) Applicant: Oridis Biomed Forschungs- und Entwicklungs GmbH, 8010 Graz (AT)
(72) Inventor: Otte, Marcus, 8010 GRAZ (AT)

(57) **Abstract**

The invention relates to compounds of formula (1) 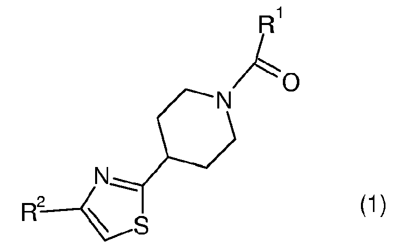 wherein R¹ represents optionally substituted amino, optionally substituted phenyl, optionally substituted heteroaryl, optionally substituted heterocyclyl, optionally substituted phenyl- or heteroaryl-alkyl, optionally substituted phenylaminocarbonyl-alkoxyalkyl or an alkenyl group as defined in the specification. These compounds are useful for the treatment of hyperproliferative diseases, in particular, liver cancers.

## Description

### Field of the invention

This invention relates to thiazole-piperidine derivates that are useful in treating of hyperproliferative diseases, e.g. cancer, preferably liver cancers such as hepatocarcinoma in mammals, especially humans. The invention also relates to the pharmaceutical compositions comprising these compounds and their use for the treatment of hyperproliferative diseases.

### Background of the invention

The international publications WO 2004/058750 and WO 2004/058751 describe thiazole-piperidine compounds, namely piperidinyl-thiazole carboxylic acid derivatives and their use in treating vascular endothelial factor (VEGF)- mediated disorders (in particular endometrosis and acute macular degenerative disorder), and piperidinyl-thiazole carboxamide derivatives and their use in altering vascular tone including myometrial and endometrial vascular tone, respectively.

Although liver cancer is relatively uncommon in the industrialized western world, it is among the leading causes of cancer worldwide. In contrast to many other types of cancer, the number of people who develop and die from liver cancer is increasing. On a global basis, primary liver cancer such as hepatocarcinoma (HCC) belongs to the most common malignant tumors accounting for about 1 million deaths/year (Bruix, J. et al., 2004, Cancer Cell (5): 215-219). The principal risk factors for liver cancer are viruses, alcohol consumption, food contamination with aflatoxin molds and metabolic disorders. Chronic hepatitis B and C virus infections are the major causes of HCC but damage from alcohol and chronic liver metabolic disorders are also recognized to result in HCC, and the mechanisms responsible for development of a tumor from these different etiologies are likely to differ. The rates of alcoholism and chronic hepatitis B and C continue to increase. The outlook therefore is for a steady increase in liver cancer rates, underscoring the need for new therapies in this area.

Primary liver cancer is difficult to treat. Surgical removal of the cancer and liver transplantation is limited to small cancers and not a viable option for most patients since at diagnosis the cancer is often in an advanced stage. Chemotherapy is occasionally used for tumors not suitable for surgery but any benefit is usually short lived. Thus, survival rates for primary liver cancer are particularly low. Conventional therapy has generally not proven effective in the management of liver cancer.

For HCC for instance, there is no effective therapeutic option except resection and transplantation but these approaches are only applicable in early stages of HCC, limited by the access to donor livers, and associated with severe risks for the patient. In addition, these approaches are extremely expensive. These cancers respond very poorly to chemotherapeutics, most likely due to the normal liver function in detoxification and export of harmful compounds. Several other therapeutic options, such as chemoembolization, cryotherapy and ethanol injection are still in an experimental phase, and the efficacy of these is not established. Thus until now no satisfactory therapies have been developed in order to be able to intervene in liver diseases and other epithelial cancers.

The spectrum of liver disease varies from mild and reversible fatty liver to progressive chronic liver disease, which results in the development of the life threatening conditions of liver cirrhosis, liver failure and liver cancer.

The major causes of chronic liver disease are infections with hepatitis B or C virus, excessive consumption of alcohol and non-alcoholic fatty liver disease (NAFLD). Hepatitis B virus (HBV) infection is a global public health issue. It is the leading cause of cirrhosis and hepatocellular carcinoma (HCC) worldwide (Conjeevaram H.S. et al., 2003, Journal of Hepatology, 38: 90-103). Hepatitis C virus (HCV), a major cause of liver-related morbidity and mortality worldwide, represents one of the main public health problems (Alberti A. and Benvegnù L., Journal of Hepatology 2003, 38: 104-118). The HCV infection frequently causes chronic hepatitis, which is linked to the development of liver cirrhosis and HCC (Cyong J.C. et al., 2002, Am J Chin Med, 28: 351-360).

Alcoholic liver disease (ALD) is the commonest cause of cirrhosis in the Western world, currently one of the ten most common causes of death. In the United States, ALD affects at least 2 million people, or approximately 1 % of the population. The true incidence of ALD, especially in its milder forms, may be substantially greater because many patients are asymptomatic and may never seek medical attention. The spectrum of ALD ranges from fatty liver (steatosis), present in most, if not all heavy drinkers, through steatohepatitis, cholestasis (characterised by blocked bile excretion from the liver), fibrosis and ultimately cirrhosis (Stewart S.F. and Day C.P, 2003, Journal of Hepatology, 38: 2-13). Although fatty liver is reversible with abstention, it is a risk factor for progression to fibrosis and cirrhosis in patients who continue drinking, particularly when steatohepatitis is present.

Non-alcoholic fatty liver disease (NAFLD) refers to a wide spectrum of liver damage, ranging from simple steatosis to steatohepatitis, cholestasis, advanced fibrosis and cirrhosis. Steatohepatitis (non-alcoholic steatohepatitis) represents only a stage within the spectrum of NAFLD (Anguilo P., 2002, New Engl. J. Med., 346: 1221-1231). The pathological picture resembles that of alcohol-induced liver injury, but it occurs in patients who do not abuse alcohol. NAFLD should be differentiated from steatosis, with or without hepatitis, resulting from secondary causes, because these conditions have distinctly different pathogens and outcomes. These secondary causes of fatty liver disease (steatosis) are nutritional (e.g. protein-calorie malnutrition, starvation, total parenteral nutrition, rapid weight loss, gastrointestinal surgery for obesity), drugs (e.g. glucocorticoids, synthetic estrogens, aspirin, calcium-channel blockers, tetracycline, valproic acid, cocaine, antiviral agents, fialuridine, interferon a, methotrexate, zidovudine), metabolic or genetic (e.g. lipodostrophy, dysbetalipoproteinemia, Weber-Christian disease, galactosaemia, glycogen storage disorders, acute fatty liver of pregnancy) and other, such as diabetes mellitus, obesity or hyperlipidaemia (Anguilo P., 2002, New Engl. J. Med., 346: 1221-1231; MacSween R.N.M. et al., 2002, Pathology of the Liver. Fourth Edition. Churchill Livingstone, Elsevier Science). Despite the prevalence of chronic liver disorders effective therapies for most disorders in this category are absent.

The severest of the non-viral chronic liver diseases, alcoholic steatohepatitis and non-alcoholic steatohepatitis (ASH and NASH), lead with high frequency to liver cirrhosis, liver failure and liver cancer (e.g. HCC). Overall, there is no proven specific treatment for ASH and NASH, having a definitive diagnosis via biopsy is not very likely to affect the management of the disease in a patient.

### Summary of the invention

The invention relates to novel compounds of formula (1), 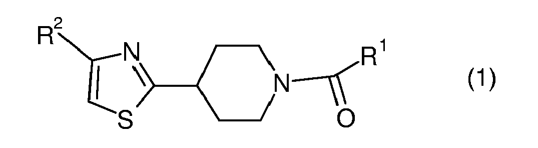 wherein
R¹ represents optionally substituted amino, optionally substituted phenyl, optionally substituted heteroaryl, optionally substituted heterocyclyl, optionally substituted phenyl- or heteroaryl-alkyl, optionally substituted phenylaminocarbonyl-alkoxyalkyl or a residue of formula (2), 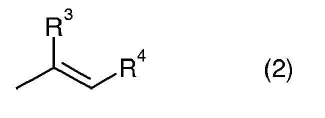 wherein R³ is hydrogen or alkyl and R⁴ is optionally substituted phenyl, optionally substituted heteroaryl, carboxy, alkoxycarbonyl, optionally substituted aminocarbonyl or cyano, and R² represents optionally substituted phenyl or optionally substituted heteroaryl; and salts thereof. The invention further relates to pharmaceutical preparations comprising these compounds and to the use of the compounds for treating hyperproliferative diseases, in particular liver cancer.

### Detailed description of the invention

The invention relates to novel compounds of formula (1), 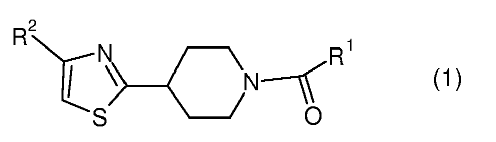 wherein
R¹ represents optionally substituted amino, optionally substituted phenyl, optionally substituted heteroaryl, optionally substituted heterocyclyl, optionally substituted phenyl- or heteroaryl-alkyl, optionally substituted phenylaminocarbonyl-alkoxyalkyl or a residue of formula (2), 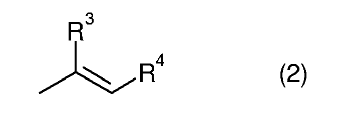 wherein R³ is hydrogen or alkyl and R⁴ is optionally substituted phenyl, optionally substituted heteroaryl, carboxy, alkoxycarbonyl, optionally substituted aminocarbonyl or cyano,
and R² represents optionally substituted phenyl or optionally substituted heteroaryl; and salts thereof.

The general terms used hereinbefore and hereinafter preferably have within the context of this disclosure the following meanings, unless otherwise indicated:

The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Double bonds in principle can have E- or Z-configuration. The compounds of this invention may therefore exist as isomeric mixtures or single isomers. If not specified both isomeric forms are intended.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula (1).

Alkyl has from 1 to 12, preferably from 1 to 10 carbon atoms, and is linear or branched. Alkyl is preferably lower alkyl, for example, C₁-C₇-alkyl, preferably C₁-C₄-alkyl .

Lower alkyl has 1 to 7 carbon atoms, preferably 1 to 4 carbon atoms, and is e.g. n-hexyl, n-pentyl, butyl, such as n-butyl, sec-butyl, isobutyl or tert-butyl, propyl, such as n-propyl or isopropyl, ethyl or methyl. Most preferably lower alkyl is methyl or ethyl.

In optionally substituted phenyl, substituents are preferably lower alkyl, lower alkoxy, lower alkoxy-lower alkoxy, methylenedioxy, 1,2-ethylene-dioxy, amino, alkylamino, acetylamino, dialkylamino, di(cyanoalkyl)amino, di(alkoxyalkyl)amino, halo-lower alkyl, lower alkoxy-lower alkyl, phenylthio-lower alkyl, phenylsulfoxy-lower alkyl, phenylsulfonyl-lower alkyl, halo, or nitro. Phenyl may be substituted by up to three of the mentioned substituents, for example as in trimethoxyphenyl. Substituents may be in ortho-, meta- or para position.

Heteroaryl represents an aromatic group containing at least one heteroatom selected from nitrogen, oxygen and sulfur, and is mono- or bicyclic. Monocyclic heteroaryl includes 5 or 6 membered heteroaryl groups containing 1, 2, 3 or 4 heteroatoms selected from nitrogen, sulfur and oxygen. Bicyclic heteroaryl includes 9 or 10 membered fused-ring heteroaryl groups. Examples of heteroaryl include pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, and benzo fused derivatives of such monocyclic heteroaryl groups, such as indolyl, benzimidazolyl, benzopyrazolyl or benzofuryl, quinolinyl, isoquinolinyl, quinazolinyl, quinoxalinyl or purinyl, preferably thienyl, pyrazolyl, imidazolyl, isoxazolyl, pyridyl, pyrazinyl, benzimidazolyl or benzopyrazolyl .

In optionally substituted heteroaryl, substituents are preferably lower alkyl, halo-lower alkyl, lower alkoxy-lower alkyl, lower alkoxy, lower alkoxy-lower alkoxy, halo-lower alkoxy, amino optionally substituted by one or two substituents lower alkyl and one substituent lower alkylcarbonyl, halo, or nitro, or another heteroaryl group of those mentioned hereinbefore, e.g. pyridyl, pyrimidinyl or pyrazinyl.

Alkenyl contains one or more, e.g. two or three, double bonds, and is preferably lower alkenyl, such as C₁-C₄-alkenyl, e.g. 1- or 2-butenyl, 1-propenyl, allyl or vinyl.

Alkinyl is preferably lower alkinyl, such as C₁-C₄-alkinyl, e.g. propargyl or acetylenyl.

In optionally substituted alkenyl or alkinyl, substituents are preferably lower alkyl, lower alkoxy, halo or di(lower alkyl)amino, and are connected with a saturated carbon atom of alkenyl or alkinyl or with an unsaturated carbon atom of alkenyl.

Heterocyclyl designates preferably a saturated, partially saturated or unsaturated, mono- or bicyclic ring containing 4-10 atoms comprising one, two or three heteroatoms selected from nitrogen, oxygen and sulfur, which may, unless otherwise specified, be carbon or nitrogen linked, wherein a ring nitrogen atom may optionally be substituted by a group selected from lower alkyl, amino-lower alkyl, aryl, aryl-lower alkyl and acyl, a ring carbon atom may be substituted by lower alkyl, amino-lower alkyl, phenyl, phenyl-lower alkyl, heteroaryl, heteroaryl-lower alkyl, lower alkoxy, hydroxy or oxo, or two ring carbons may be annulated by a benzo group. Examples of heterocyclyl are pyrrolidinyl, oxazolidinyl, thiazolidinyl, piperidinyl, morpholinyl, piperazinyl, dioxolanyl and tetrahydropyranyl.

Acyl designates, for example, alkylcarbonyl, cyclohexylcarbonyl, arylcarbonyl, aryl-lower alkylcarbonyl, or heteroarylcarbonyl. Lower acyl is preferably C₁-C₄-alkylcarbonyl, in particular propionyl or acetyl.

Hydroxyalkyl is especially hydroxy-lower alkyl, preferably hydroxy-C₁-C₄-alkyl, e.g. hydroxymethyl, 2-hydroxyethyl or 2-hydroxy-2-propyl.

Haloalkyl is especially halo-lower alkyl, preferably halo-C₁-C₄-alkyl, e.g. fluoroalkyl, especially trifluoromethyl, pentafluoroethyl or 3,3,3-trifluoroethyl.

Halogen is fluorine, chlorine, bromine or iodine.

Lower alkoxy is especially C₁-C₄-alkoxy, e.g. methoxy, ethoxy, isopropyloxy, or tert-butyloxy.

Phenylalkyl includes phenyl and alkyl as defined hereinbefore, and is e.g. benzyl, 1-phenethyl or 2-phenethyl.

Heteroarylalkyl includes heteroaryl and alkyl as defined hereinbefore, and is e.g. 2-pyridyl-methyl, 3-pyridyl-methyl, 4-pyridyl-methyl, 1- or 2-pyrrolyl-methyl, 1-imidazolyl-methyl, 2-(1-imidazolyl)-ethyl or 3-(1-imidazolyl)-propyl.

In substituted amino, the substituents are preferably lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, lower alkylcarbonyl, lower alkylcarbonyl-lower alkyl, cyano-lower alkyl, or the two substituents on nitrogen form together with the nitrogen heteroaryl or heterocyclyl. In particular, substituted amino is C₁-C₄-alkylamino, di-C₁-C₄-alkylamino, or C₁-C₄-alkoxycarbonyl-C₁-C₇-alkylamino.

In optionally substituted phenylaminocarbonyl-alkoxyalkyl the substituent are those mentioned above under optionally substituted phenyl, in particular lower alkyl and halo. In particular, optionally substituted phenylaminocarbonyl-alkoxyalkyl is halo- and C₁-C₄-alkyl substituted phenylaminocarbonyl-C₁-C₄-alkoxy-C₁-C₄-alkyl.

Salts are especially the pharmaceutically acceptable salts of compounds of formula (1).

Such salts are formed, for example, as acid addition salts, preferably with organic or inorganic acids, from compounds of formula (1) with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methyl maleic acid, cyclohexanecarboxylic acid, adamantine-carboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1 ,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

The compound of the formula (1) may be administered in the form of a pro-drug which is broken down in the human or animal body to give a compound of the formula (1).

Examples of pro-drugs include in vivo hydrolysable esters of a compound of the formula (1).

The compounds of formula (1) have valuable pharmacological properties. The invention also relates to compounds of formula (1) as defined hereinbefore for use as medicaments.

The efficacy of the compounds of the invention in inhibition of proliferation of tumor cell lines can be demonstrated as follows: To determine the inhibition of cell proliferation a luminescent cell viability assay (CellTiter-Glo^{®}; Promega, Madison, USA) is implemented. The assay is based on the relation that the ATP content is directly proportional to the number of cells present in the culture. The CellTiter-Glo^{®} assay is performed to measure the proliferation rate of human hepatoma (HuH7, PLC/PRF/5, Hep3B, SK-Hep1), human hepatoblastoma (HepG2), human colon carcinoma (CX-2), human breast cancer (SK-Br-3), human ovarian carcinoma (HeLa) and human pancreatic carcinoma (DAN-G) cell lines (see Example 2). On the basis of these studies a compound of formula (1) according to the invention shows therapeutic efficacy especially against cancer, preferably in liver and pancreas. In particular, E-3-{4-[4-(3,4-ethylendioxphenyl)-thiazol-2-yl]-piperidin-1-yl}-1-(3,4-methylendioxyphenyl)-3-oxo-prop-1-ene (compound no. I in Table 1 / Example 2) blocks the proliferation of HuH7 with an EC50 of 0.33 µM. Further, HepG2 and Hep3B are inhibited with an EC50 of 0.9 and 0.3 µM, respectively. Compounds of the formula (1) show inhibition of cell proliferation of other tumor cell lines (Table 2 in Example 2), thus suggesting to be useful for treatment of hyperpoliferative diseases, such as cancers.

Furthermore, to assess in vivo activity of potential anti-proliferative drug candidates' standard in vivo models of liver tumors, e.g. the ectopic (subcutaneous) and orthotopic transplantation of human tumor cells in immune deficient mice like BALB/c nude or SCID mice can be used. Tumor type specificity is generated by the selection of cell lines for transplantation. In general the subcutaneously transplantation of human tumor cell lines is a general model to assess the in vivo activity of potential anti-proliferative drug candidates. Further, orthotopic transplantation models offer the ability to analyse the tumor in its typical micro environment (Armengol C. et al., Clin. Can. Res. 2004, 10: 2150-2157).
For the subcutaneous transplantation (ectopic transplantation) of human liver tumor cells (i.e. HuH7, PLC/PRF/5, Hep3B, SK-Hep1 and HepG2) 1 to 5 million cells are subcutaneously inoculated into the left flank of male BALC/nude mice (Yeo E-J. et al., J. Natl. Cancer Inst. 2003, 95: 516-525). Tumor formation can be supported by inoculation of a tumor cells suspension in extracellular matrix proteins like for example matrigel (10 mg/ml; BD biosciences). Typically tumor formation could be observed after two to four weeks, dependent on the type of tumor cells and support medium used. Treatment start could be either (1) a few days (typically 2-5) after tumor cell inoculation, or (2) after a certain tumor size (250 to 450 mm³) has been reached. The time point of treatment start allows distinguishing between inhibition of tumor formation (1) and reduction of tumor mass (2). Statistical analysis comparing tumor growth curves, tumor mass and tumor volume of serum versus placebo treated groups are finally performed.

Transplantation of human liver tumor cells into the mouse liver of immune deficient mice or mouse liver tumor cells in immune competent male mouse strains allow not only to compare tumor growth characteristics in treated and non-treated animals but also to compare survival time.

Further the DEN (N-nitroso-di-ethylamin) induced growth of liver tumors, supported by the administration of phenobarbital in C3H/He mice could be used to test the activity of potential drug candidates against liver tumors (Kemp C. et al., Proc. Natl. Acad. Sci. USA 1989, 86: 7505-7509 and Shiota G. et al., Carcinogenesis 1999, 20: 59-63). Male C3H/He mice are intoxicated with a single dose of DEN at age 6-8 weeks. Feeding of phenobarbital starts one week after DEN intoxication and is continued for 40 weeks. Huge enlargement of the liver is palpable even earlier. Typically the liver is enlarged around 5 to 6 times compared to non-treated mice. Liver tumors are macroscopically and microscopically detectable.

In particular, the compounds of the invention are active in regard of hyperproliferative, inflammatory, autoimmune and metabolic diseases of liver, pancreas and of any other organ such as breast, colon, lung, prostate, ovary, kidney.

The term "hyperproliferative diseases" includes neoplastic diseases, preferably against malignancies, e.g. epithelial neoplasms (cancer). The term "epithelial cancer" within the meaning of the invention includes carcinomas of any organ, preferably of the liver (such as hepatocarcinoma), pancreas (e.g. adenocarcinoma of the pancreas), lung, stomach, colon, prostate, kidney, skin and breast, and refers to disorders of these organs in which epithelial cell components of the tissue are transformed resulting in a malignant tumor identified according to the standard diagnostic procedures as generally known to a person skilled in the art. Furthermore the term "hyperpoliferative disease" includes squamous cell neoplasms, basal cell neoplasms, transitional cell papillomas and carcinomas, adenocarcinomas, adnexal and skin appendage neoplasms, mucoepidermoid neoplasms, cystic neoplasms, mucinous and serous neoplasms, ductal-, lobular and medullary neoplasms, acinar cell neoplasms, complex epithelial neoplasms, specialized gonadal neoplasms, paragangliomas and glomus tumors, melanomas, soft tissue tumors and sarcomas, fibromatous neoplasms, myxomatous neoplasms, lipomatous neoplasms, myomatous neoplasms, complex mixed and stromal neoplasms, fibroepithelial neoplasms, synovial like neoplasms, mesothelial neoplasms, germ cell neoplasms, trophoblastic neoplasms, mesonephromas, blood vessel tumors, lymphatic vessel tumors, osseous and chondromatous neoplasms, giant cell tumors, miscellaneous bone tumors, odontogenic tumors, gliomas, neuroepitheliomatous neoplasms, meningiomas, nerve sheath tumors, granular cell tumors and alveolar soft part sarcomas, Hodgkin's and non Hodgkin's lymphomas, other lymphoreticular neoplasms, plasma cell tumors, mast cell tumors, immunoproliferative diseases, leukemias, miscellaneous myeloproliferative disorders, lymphoproliferative disorders and myelodysplastic syndromes. "Hyperproliferative disease" also refers to benign hyperproliferative diseases such as epithelial tumors e.g. adenoma (in particular focal nodular hyperplasia), pancreatic islet cell adenoma, pancreatic adenoma and pancreatic oedema, and other tumors derived from mesenchymal tissue, such as fibroma, lipoma, and naevi.

The term "inflammatory diseases" represents diseases of various organs caused by viruses, bacteria, fungi, mycoplasms or other microorganisms, in particular inflammatory diseases of liver, such as viral and non-viral hepatitis (e.g. hepatitis B and hepatitis C), and of pancreas, such as chronic or acute pancreatitis. The inflammatory diseases refer to inflammation caused by physiological byproducts of metabolism, such as cholangitis caused by bile acids, or arthritis.

The compounds of the invention are likewise active in autoimmune diseases. The term "autoimmune diseases" refers to e.g. systemic, discoid or subacute cutaneous lupus erythematosus, rheumatoid arthritis, antiphospholipid syndrome, CREST, progressive systemic sclerosis, mixed connective tissue disease (Sharp syndrome), Reiter's syndrome, juvenile arthritis, cold agglutinin disease, essential mixed cryoglobulinemia, rheumatic fever, ankylosing spondylitis, chronic polyarthritis, myasthenia gravis, multiple sclerosis, chronic inflammatory demyelinating polyneuropathy, Guillan-Barre syndrome, dermatomyositis / polymyositis, autoimmune hemolytic anemia, thrompocytopenic purpura, neutropenia, type I diabetes mellitus, thyroiditis (including Hashimoto's and Grave' disease), Addison's disease, polyglandular syndrome, pemphigus (vulgaris, foliaceus, sebaceous and vegetans), bullous and cicatricial pemphigoid, pemphigoid gestationis, epidermolysis bullosa acquisita, linear IgA disease, lichen sclerosus et atrophicus, morbus Duhring, psoriasis vulgaris, guttate, generalized pustular and localized pustular psoriasis, vitiligo, alopecia areata, primary biliary cirrhosis, autoimmune hepatitis, all forms of glomerulonephritis, pulmonal hemorrhage (goodpasture syndrome), IgA nephropathy, pernicious anemia and autoimmune gastritis, inflammatory bowel diseases (including colitis ulcerosa and morbus Crohn), Behcet's disease, Celic-Sprue disease, autoimmune uveitis, autoimmune myocarditis, granulomatous orchitis, aspermatogenesis without orchitis, idiopatic and secondary pulmonary fibrosis, inflammatory diseases with a possibility of autoimmune pathogensesis, such as pyoderma gangrensosum, lichen ruber, sarcoidosis (including Löfgren and cutaneous/subcutaneous type), granuloma anulare, allergic type I and type IV immunolgical reaction, asthma bronchiale, pollinosis, atopic, contact and airborne dermatitis, large vessel vasculitis (giant cell and Takayasu's arteritis), medium sized vessel vasculitis (polyarteritis nodosa, Kawasaki disease), small vessel vasculitis (Wegener's granulomatosis, Churg Strauss syndrome, microscopic polangiitis, Henoch-Schoenlein purpura, essential cryoglobulinemic vasculitis, cutaneous leukoklastic angiitis), hypersensitivity syndromes, toxic epidermal necrolysis (Stevens-Johnson syndrome, erythema multiforme), diseases due to drug side effects, all forms of cutaneous, organ-specific and systemic effects due to type ├VI (Coombs classification) immunologic forms of reaction, transplantation related pathologies, such as acute and chronic graft versus host and host versus graft disease, involving all organs (skin, heart, kidney, bone marrow, eye, liver, spleen, lung, muscle, central and peripheral nerve system, connective tissue, bone, blood and lymphatic vessel, genito-urinary system, ear, cartilage, primary and secondary lymphatic system including bone marrow, lymph node, thymus, gastro-intestinal tract, including oro-pharynx, esophageus, stomach, small intestine, colon, and rectum, including parts of above mentioned organs down to single cell level and substructures, e.g. stem cells).

The term "metabolic diseases" includes conditions in which there is a deviation from a metabolic process or which is caused by an abnormal metabolic process. Metabolic disease can be congenital due to inherited enzyme abnormality or acquired due to disease of an endocrine organ or failure of a metabolically important organ, preferably of the liver (such as liver cirrhosis).

Furthermore, the compounds of the invention are active in liver diseases.

The term "liver disease" refers to and comprises all kinds of diseases that preferably affect the anatomy, physiology, metabolic, and/or genetic activities of the liver, that preferably affect the generation of new liver cells, and/or the regeneration of the liver, as a whole or parts thereof, preferably transiently, temporarily, chronically or permanently in a pathological way. Preferably also included are inherited liver diseases and neoplastic liver diseases. Within the meaning of the present invention the term "liver disease" preferably also encompasses liver cancer, in particular hepatocellular carcinoma (HCC), benign liver neoplasms such as adenoma and/or FNH. Preferably HCC further comprises subtypes of the mentioned disorders, including liver cancers characterized by intracellular proteinaceous inclusion bodies, HCCs characterized by hepatocyte steatosis, and fibrolamellar HCC. For example, precancerous lesions are preferably also included such as those characterized by increased hepatocyte cell size (the "large cell" change), and those characterized by decreased hepatocyte cell size (the "small cell" change) as well as macro regenerative (hyperplastic) nodules (Anthony, P. in MacSween et al., eds. Pathology of the Liver. 2001, Churchill Livingstone, Edinburgh). Liver disease is further understood to preferably comprise liver diseases caused by trauma, intoxication, in particular by alcohol, drugs or food intoxication, radiation, infection, cholestasis, immune reactions, insulin resistance, diabetes and by inherited metabolic liver diseases. Preferred examples of liver diseases include cirrhosis, alcoholic liver disease, chronic hepatitis, Wilson's Disease, and heamochromatosis. Preferably further included are autoimmune diseases of liver.

The compounds of the invention are also active in pancreas diseases.

"Pancreas diseases" include all kinds of diseases that preferably affect the anatomy, physiology, metabolic, and/or genetic activities of the pancreas, preferably tumors (such as adenocarcinoma of pancreas), inflammatory diseases of pancreas (e.g. acute pancreatitis and abscess), or adverse reactions in context of pancreatic transplantations, such as graft pancreatitis and pancreatic trombosis (Rosai and Ackerman's Surgical PathologyPathology, 9th edition, Volume one, Mosby, Chapter 15, pg. 1061-1115).

Preferred embodiment for the use of compounds of formula (1) is the treatment of hyperproliferative disorders of the liver such as liver cancer (in particular HCC), focal nodular hyperplasis (FNH), viral and non-viral hepatitis and liver cirrhosis.

Another preferred embodiment is the treatment of hyperproliferative disorders of the pancreas like pancreas adenocarcinoma, pancreatic adenoma, pancreatic islet cell adenoma and carcinoma, by using the compounds according to the invention.

Another preferred embodiment is the treatment of other hyperproliferative, malignant transformation typically characterized by increasing genomic alterations during disease progression like gene translocation, gene amplification, loss of heterozygocity and telomere shortening as for example carcinomas of liver, pancreas, colon, lung, breast, prostate and other organs such as ovary, uterus, kidney. Furthermore, there are included leukemias such as acute leukemia, acute panmyeolosis, myeloid sarcoma, myelodysplastic syndromes, chronic myeloid leukemia, polycythemia vera, chronic lymphocytic leukemia, prolymphocytic leukemia, hairy cell leukemia, plasma cell leukaemia; lymphomas (e.g. B-cell lymphoma, T-cell lymphoma, Hodgkin's lymphoma, lymphoplasmacytic lymphoma); myeolomas such as plasma cell myeloma and osteosclerotic myeloma; sarcomas including osteosarcoma, chodrosarcoma, giant cell tumors, fibrosarcoma, liposarcoma, angiosarcoma, lymphangiosarcoma, leiomyosarcoma, rhabdomyosarcoma, celar celll sarcoma, epitheloid sarcoma, Ewing's sarcoma; and melanomas.

A further preferred embodiment is the treatment of benign and semi-malignant hyperproliferative diseases like psoriasis and basalioma by compounds according to the invention.

Compounds of formula (1) are particularly valuable in treating hyperproliferative diseases.

Furthermore, compounds of formula (1) are more particularly valuable in treating liver cancers.

Compounds of formula (1) are even more particularly valuable in treating hepatocarcinoma.

The invention further comprises the use of compounds of formula (1) for the preparation of a medicament for treating hyperproliferative diseases, in particular those that are listed hereinbefore.

A compound of formula (1) can be administered alone or in combination with one or more other therapeutic agents, possible combination therapy taking the form of fixed combinations, or the administration of a compound of the invention and one or more other therapeutic agents being staggered or given independently of one another, or the combined administration of fixed combinations and one or more other therapeutic agents. A compound of formula (1) can, besides or in addition, be administered especially for tumor therapy in combination with chemotherapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk. Particularly preferred is the use of compounds of formula (1) in combination with chemotherapy.

Therapeutic agents for possible combination are especially one or more cytostatic or cytotoxic compounds, for example a chemotherapeutic agent or several selected from the group comprising indarubicin, cytarabine, interferon, hydroxyurea, bisulfan, or an inhibitor of polyamine biosynthesis, an inhibitor of protein kinase, especially of serine/threonine protein kinase, such as protein kinase C, or of tyrosine protein kinase, such as epidermal growth factor receptor tyrosine kinase, a cytokine, a negative growth regulator, such as TGF-β or IFN-β, an aromatase inhibitor, a classical cytostatic, an inhibitor of the interaction of an SH2 domain with a phosphorylated protein, an inhibitor of Bcl-2 and modulators of the Bcl-2 family members such as Bax, Bid, Bad, Bim, Nip3 and BH3-only proteins.

A compound according to the invention is not only for the (prophylactic and preferably therapeutic) management of humans, but also for the treatment of other warm-blooded animals, for example of commercially useful animals, for example rodents, such as mice, rabbits or rats, or guinea-pigs. Such a compound may also be used as a reference standard in the test systems described above to permit a comparison with other compounds.

With the groups of preferred compounds of formula (1) mentioned hereinafter, definitions of substituents from the general definitions mentioned hereinbefore may reasonably be used, for example, to replace more general definitions with more specific definitions or especially with definitions characterized as being preferred.

In particular, the invention refers to compounds of formula (1) wherein R¹ is optionally substituted amino, for example amino substituted by lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, lower alkoxycarbonyl, lower alkoxycarbonyl-lower alkyl, cyano-lower alkyl, or wherein the two substituents on nitrogen form together with the nitrogen heteroaryl or heterocyclyl. In a preferred embodiment, the invention refers to compounds of formula (1) wherein R¹ is C₁-C₄-alkylamino, di-C₁-C₄-alkylamino or C₁-C₄-alkoxycarbonyl-C₁-C₇-alkylamino, in particular C₁-C₄-alkoxycarbonyl-C₁-C₇-alkylamino.

In another embodiment, the invention refers to compounds of formula (1) wherein R¹ is optionally substituted phenyl, for example phenyl substituted by lower alkyl, lower alkoxy, lower alkoxy-lower alkoxy, methylenedioxy, 1,2-ethylene-dioxy, amino, alkylamino, acetylamino, dialkylamino, di(cyanoalkyl)amino, di(alkoxyalkyl)amino, halo-lower alkyl, lower alkoxy-lower alkyl, phenylthio-lower alkyl, phenylsulfoxy-lower alkyl, phenylsulfonyl-lower alkyl, halo, or nitro, in particular phenyl with up to three of the mentioned substituents, for example as in trimethoxyphenyl. Substituents may be in ortho-, meta- or para position. Also preferred as substituted phenyl is methylenedioxyphenyl, 1,2-ethylenedioxyphenyl, bromophenyl, fluorophenyl, di-C₁-C₄-alkylaminophenyl, di(cyano-C₁-C₄-alkyl)amino and phenylsulfoxy-C₁-C₄-alkyl, in particular di(cyano-C₁-C₄-alkyl)amino and phenylsulfoxy-C₁-C₄-alkyl.

In another embodiment, the invention refers to compounds of formula (1) wherein R¹ is optionally substituted heteroaryl, for example pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, indolyl, benzimidazolyl, benzopyrazolyl, benzofuryl, quinolinyl, isoquinolinyl, quinazolinyl, quinoxalinyl or purinyl, preferably thienyl, pyrazolyl, imidazolyl, isoxazolyl, thiazolyl, pyridyl, pyrazinyl, benzimidazolyl or benzopyrazolyl, optionally substituted by lower alkyl, halo-lower alkyl, lower alkoxy-lower alkyl, lower alkoxy, lower alkoxy-lower alkoxy, halo-lower alkoxy, amino optionally substituted by one or two substituents lower alkyl and one substituent lower alkylcarbonyl, halo, or nitro, or another heteroaryl group of those mentioned hereinbefore, e.g. pyridyl, pyrimidinyl or pyrazinyl. Preferred are compounds of formula (1) wherein R¹ is optionally substituted pyridyl, thiazolyl or benzopyrazolyl, in particular fluoro-C₁-C₄-alkoxy-pyridyl, such as 2-(2,2,2-trifluoroethoxy)-3-pyridyl, pyrazinyl-thiazolyl, such as 2-pyrazinyl-4-thiazolyl, or 3-benzopyrazolyl.

In yet another embodiment, the invention refers to compounds of formula (1) wherein R¹ is heterocyclyl, in particular pyrrolidinyl, oxazolidinyl, thiazolidinyl, piperidinyl, morpholinyl, piperazinyl, dioxolanyl and tetrahydropyranyl, optionally substituted at a ring nitrogen atom by a group selected from lower alkyl, amino-lower alkyl, aryl, aryl-lower alkyl and acyl, at a ring carbon atom by lower alkyl, amino-lower alkyl, phenyl, phenyl-lower alkyl, heteroaryl, heteroaryl-lower alkyl, lower alkoxy, hydroxy or oxo, or on two ring carbons by a benzo group. Preferred are compounds of formula (1) wherein R¹ is optionally substituted pyrrolidinyl or dioxolanyl, in particular oxo-N-(heteraryl-alkyl)-pyrrolidinyl, such as 2-oxo-N-(2-thienylmethyl)-4-pyrrolidinyl, or benzo-1,4-dioxolanyl.

In yet another embodiment, the invention refers to compounds of formula (1) wherein R¹ is optionally substituted phenylalkyl or optionally substituted heteroarylalkyl, in particular phenyl-C₁-C₄-alkyl with a halo substituent on phenyl and an amino substituent on alkyl, preferably fluorophenyl-acetylamino-C₁-C₄-alkyl, such as 2-(2-fluorophenyl)-1-(acetylamino)-ethyl, or benzimidazolyl-C₁-C₄-alkyl, such as 3-(2-benzimidazolyl)-propyl.

In yet another embodiment, the invention refers to compounds of formula (1) wherein R¹ is optionally substituted phenylaminocarbonyl-alkoxyalkyl, in particular wherein the substituents are lower alkyl and halo, preferably halo- and C₁-C₄-alkyl substituted phenylaminocarbonyl-C₁-C₄-alkoxy-C₁-C₄-alkyl, such as 4-chloro-2-methylphenylamino-carbonylmethoxymethyl.

In yet another embodiment, the invention refers to compounds of formula (1) wherein R¹ is a residue of formula (2), 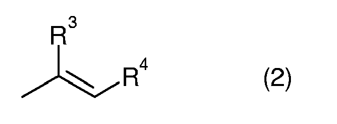 wherein R³ is hydrogen or alkyl and R⁴ is optionally substituted phenyl, optionally substituted heteroaryl, carboxy, alkoxycarbonyl, optionally substituted aminocarbonyl or cyano. Preferred are compounds wherein R¹ is a residue of formula (2) and R³ is hydrogen or C₁-C₄-alkyl and R⁴ is optionally substituted phenyl, optionally substituted pyrazolyl, or C₁-C₄-alkoxycarbonyl. Particularly preferred are compounds wherein R¹ is a residue of formula (2) and R³ is hydrogen or methyl and R⁴ is trimethoxyphenyl, methylenedioxyphenyl, pyrazolyl substituted by methyl and halo, or C₁-C₄-alkoxycarbonyl.

In another preferred embodiment R² represents optionally substituted phenyl, in particular phenyl substituted by halo, nitro, dialkylamino and/or ethylenedioxy, in particular 4-chloro-3-nitrophenyl, 3-bromophenyl, 4-diethylaminophenyl or 3,4-ethylenedioxyphenyl.

In yet another preferred embodiment R² represents optionally substituted heteroaryl, in particular substituted isoxazolyl, such as alkoxycarbonyl-isoxazolyl, e.g. 3-ethoxycarbonyl-5-isoxazolyl.

Preferred are those compounds wherein both R¹ and R² have one of the preferred meaning listed above, and most preferred are the compounds of the Example 2 (Table 1A to 1 D), especially the compound of formula (1) wherein R¹ is a residue of formula (2), R² is 3,4-ethylenedioxyphenyl, R³ is hydrogen and R⁴ is 3,4-methylenedioxyphenyl; the compound of formula (1) wherein R¹ is a residue of formula (2), R² is 4-chloro-3-nitrophenyl, R³ is hydrogen and R⁴ is ethoxycarbonyl; the compound of formula (1) wherein R¹ is 3-(1 H-benzimidazol-2-yl)-propyl and R² is 3-bromo-phenyl; and the compound of formula (1) wherein R¹ is 2-(2-fluorophenyl)-1-(acetylamino)-ethyl and R² is 3-ethoxycarbonyl-isoxazol-5-yl (compounds No. I, XVII, X and III in Example 1 and 2 / Tables 1 A to 1D and Table 2).

The most preferred embodiment is the compound of formula (1)
wherein R¹ is a residue of formula (2), R² is 3,4-ethylenedioxyphenyl bound to the carbon atom of the thiazole ring adjacent to nitrogen, R³ is hydrogen and R⁴ is 3,4-methylenedioxyphenyl.

Especially, the invention relates to the use of a compound of formula (1), a prodrug or a pharmaceutically acceptable salt of such a compound for the preparation of a pharmaceutical composition for the treatment of a hyperproliferative disease.

Furthermore, the invention provides a method for the treatment of a hyperproliferative disease, which comprises administering a compound of formula (1), a prodrug or a pharmaceutically acceptable salt thereof, wherein the radicals and symbols have the meanings as defined above, in a quantity effective against said disease, to a warm-blooded animal requiring such treatment.

### Method of preparation

A compound of the invention may be prepared by processes that, though not applied hitherto for the new compounds of the present invention, are known *per se*, in particular the reaction of an a-bromoketone of formula (3), 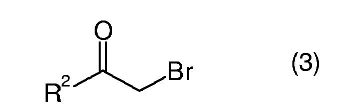 wherein reactive functional groups are optionally protected, with a thiocarboxamide of formula (4), 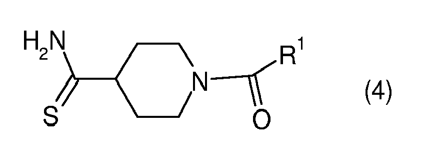 wherein reactive functional groups are optionally protected, in the presence of an inert base, optionally deprotecting protected functional groups and optionally further processing substituents R¹ and R² to the desired structure. The starting compounds of formula (3) and (4) are known or can be synthesized in analogy to known processes. In a variant of the process described hereinbefore, the residue R¹CO in formula (4) is a protecting group, e.g. tert-butoxycarbonyl, which can be eliminated by acid treatment to yield the free piperidine (H in place of residue R¹CO) and thereafter be replaced by another residue R¹CO by condensation with the corresponding acid R¹COOH or a reactive derivative thereof under conditions well known in the art, e.g. as used in peptide synthesis.

The protection of functional groups by protecting groups, the protecting groups themselves, and their removal reactions are described, for example, in standard reference books on protective groups such as T.W. Greene and P.G.M. Wuts, "Protective Groups in Organic Synthesis", Wiley, 3rd edition 1999.

All process steps described here can be carried out under known reaction conditions, in the absence of or usually in the presence of solvents or diluents, preferably such as are inert to the reagents used and able to dissolve these, in the absence or presence of catalysts, condensing agents or neutralising agents, depending on the type of reaction and/or reactants at reduced, normal, or elevated temperature, for example in the range from -100°C to about 190°C, preferably from about -80°C to about 150°C, for example at -80°C to +60 °C, at -20°C to +40°C, at room temperature, or at the boiling point of the solvent used, under atmospheric pressure or in a closed vessel, where appropriate under pressure, and/or in an inert atmosphere, for example under argon or nitrogen.

### Pharmaceutical preparations, methods, and uses

The present invention relates also to pharmaceutical compositions that comprise a compound of formula (1) as active ingredient and that can be used especially in the treatment of the diseases mentioned at the beginning. Compositions for enteral administration, such as nasal, buccal, rectal or, especially, oral administration, and for parenteral administration, such as intravenous (e.g. chemoembolization and continuous infusion), intramuscular or subcutaneous administration, to warm-blooded animals, especially humans, are especially preferred. The compositions comprise the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The present invention relates especially to pharmaceutical compositions that comprise a compound of formula (1), a tautomer, a prodrug or a pharmaceutically acceptable salt, or a hydrate or solvate thereof, and at least one pharmaceutically acceptable carrier.

The invention relates also to pharmaceutical compositions for use in a method for the prophylactic or especially therapeutic management of the human or animal body, in particular in a method of treating neoplastic disease, inflammatory disease, autoimmune disease, metabolic disease, transplantation related pathology and/or degenerative disease, especially those mentioned hereinabove.

The invention relates also to processes and to the use of compounds of formula (1) thereof for the preparation of pharmaceutical preparations which comprise compounds of formula (1) as active component (active ingredient).

A pharmaceutical composition for the prophylactic or especially therapeutic management of a neoplastic disease, autoimmune disease, transplantation related pathology and/or degenerative disease, of a warm-blooded animal, especially a human or a commercially useful mammal requiring such treatment, comprising a novel compound of formula (1) as active ingredient in a quantity that is prophylactically or especially therapeutically active against the said diseases, is likewise preferred.

The pharmaceutical compositions comprise from approximately 1 % to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

The pharmaceutical compositions of the present invention are prepared in a manner known *per se*, for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredient alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known *per se*, for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80^{®} (polyoxyethylene(20)sorbitan mono-oleate).

Suspensions in oil comprise as the oil component the vegetable, synthetic, or semisynthetic oils customary for injection purposes. In respect of such, special mention may be made of liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol. As mixtures of fatty acid esters, vegetable oils such as cottonseed oil, almond oil, olive oil, castor oil, sesame oil, soybean oil and groundnut oil are especially useful.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or microbicides, such as sorbic acid or benzoic acid.

The present invention relates furthermore to a method for the treatment of a neoplastic disease, inflammatory disease, autoimmune disease, metabolic disease, transplantation related pathology and/or degenerative disease, which comprises administering a compound of formula (1) or a pharmaceutically acceptable salt thereof, wherein the radicals and symbols have the meanings as defined above for formula (1), in a quantity effective against said disease, to a warm-blooded animal requiring such treatment. The compounds of formula (1) can be administered as such or especially in the form of pharmaceutical compositions, prophylactically or therapeutically, preferably in an amount effective against the said diseases, to a warm-blooded animal, for example a human, requiring such treatment. In the case of an individual having a bodyweight of about 70 kg the daily dose administered is from approximately 0.05 g to approximately 5 g, preferably from approximately 0.25 g to approximately 1.5 g, of a compound of the present invention.

The present invention relates especially also to the use of a compound of formula (1), or a pharmaceutically acceptable salt thereof, especially a compound of formula (1) which is said to be preferred, or a pharmaceutically acceptable salt thereof, as such or in the form of a pharmaceutical formulation with at least one pharmaceutically acceptable carrier for the therapeutic and also prophylactic management of one or more of the diseases mentioned hereinabove, in particular a neoplastic disease, autoimmune disease, transplantation related pathology and/or degenerative disease.

The preferred dose quantity, composition, and preparation of pharmaceutical formulations (medicines) which are to be used in each case are described above.

The following Examples serve to illustrate the invention without limiting the invention in its scope.

### Examples

Abbreviations: eq: Equivalent(s); DMF: Dimethylformamide; RT: Room temperature; ON: Over night; TEA: Triethylamine; DCM: Dichloromethane; TFA: Trifluoroacidic acid; MeCN: Acetonitrile; DIPEA: Diisopropylethylamine; HBTU:2-(1H-benzotriazole-1-yl)-1,1,3,3-tetramethyluronium hexafluorophosphate; TLC: Thin layer chromatography

### Example 1: Preparation of E-3-{4-[4-(3,4-ethylendioxphenyl)-thiazol-2-yl]-piperidin-1-yl}-1-(3,4-methylendioxyphenyl)-3-prop-1-ene (Compound I)

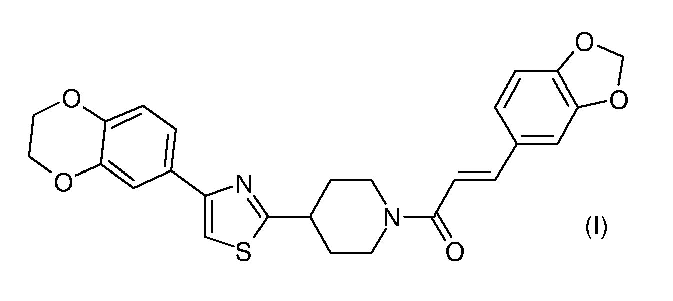

### A. Preparation of intermediate (7)

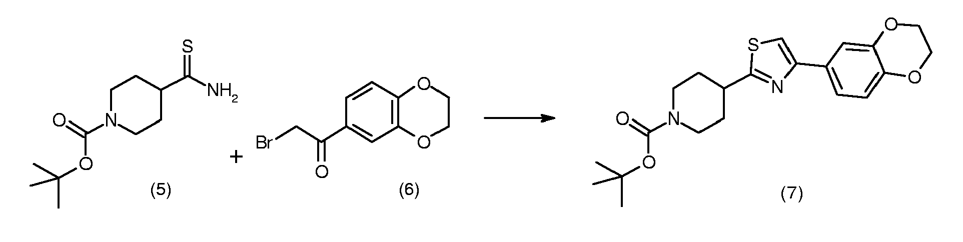

To a solution of tert-butyl 4-(aminocarbonothioyl)piperidine-1-carboxylate (5) (1 eq.) in DMF (5 vol eq.) a solution of bromoketone (6) (1.05 eq.) in DMF (10 vol eq.) is added drop wise at 0°C. The mixture is left to stir overnight gradually warming to room temperature. Triethylamine (2.2 eq.) is added portionwise to the resulting solution at room temperature, and then stirred for further 30 minutes. The reaction mixture is poured into water and extracted 3 times with ethyl acetate. The organic layers are combined, washed with waterand brine, dried overnight over MgSO₄, and concentrated in vacuo. The desired product is obtained as a pale orange oily residue, collected and dried in a vacuum oven. Yield 68%.

### B. Deprotection of intermediate (7)

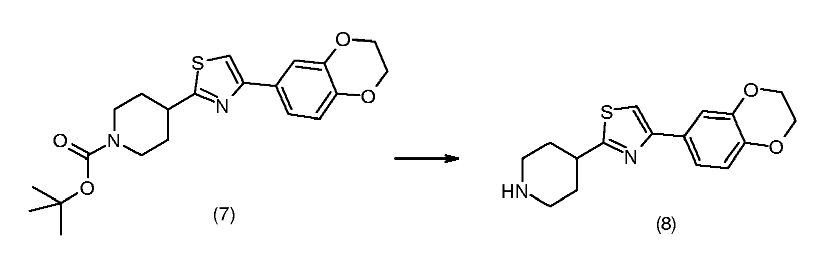

This reaction is carried out under an atmosphere of nitrogen. To a solution of tert-butoxycarbonyl protected piperidine (7) (1 eq.) in DCM (10 vol eq.) at 0°C is added TFA (10 vol eq.) drop wise. Once the addition is finished the reaction mixture is stirred at this temperature for further 10 minutes. The ice bath is removed, and the reaction mixture is stirred at room temperature over night. The reaction mixture is poured into a saturated solution of sodium carbonate; and ice is added to keep the temperature at about 10°C. The organic phase is separated, and the basic aqueous solution is extracted 3 times with DCM. The organic layers are combined, washed with water and brine, and dried over MgSO₄, and the solvent concentrated to form a solid. Product (8) is collected and dried, yield 78%.

### C. Introduction of R¹

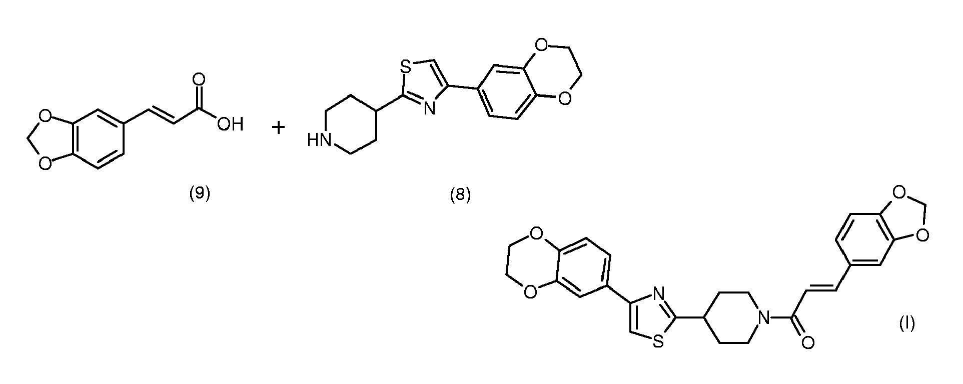

To a suspension of piperidine (8) (1 eq.), 3-(1,3-benzodioxol-5-yl)acrylic acid (9) (0.95 eq.) and HBTU (1 eq.) in acetonitile 14 (22 ml/g) under nitrogen at room temperature DIPEA (3 eq.) is added drop wise over 30 min. Once the addition is finished, the reaction mixture is stirred over night at room temperature. TLC (ethyl acetate) suggested that the reaction is completed. The solvent is concentrated to obtain an oily residue. It is dissolved in ethyl acetate, washed with sodium carbonate saturated solution, water and brine, and dried over MgSO₄; the solvent is concentrated to an oily residue. The crude product is purified using column chromatography, eluted with ether. Fractions containing pure product are combined, concentrated to obtain a white solid, collected and dried to afford the desired product compound (I) in 55%yield.

### Example 2: Proliferation assay

To determine the inhibition of cell proliferation a luminescent cell viability assay (CellTiter-Glo^{®}; Promega, Madison, USA) is implemented. The assay is based on the relation that the ATP content is directly proportional to the number of cells present in the culture, see Technical Bulletin No. 228 "CellTiter-Glo^{®} Luminescent Cell viability Assay", Revised 2/04. Promeg, Madison, USA. The CellTiter-Glo^{®} assay is performed to measure the proliferation rate of human hepatoma (HuH7, PLC/PRF/5, Hep3B, SK-Hep1), human hepatoblastoma (HepG2), human colon carcinoma (CX-2), human breast cancer (SK-Br-3), human ovarian carcinoma (HeLa) and human pancreatic carcinoma (DAN-G) cell lines. 3000 viable cells (viability ≥ 90%) are seeded in opaque-walled 96-well plates and incubated for 24 hours in DMEM, supplemented with 2 mM glutamin. Cell proliferation is stimulated by addition of FCS to a final concentration of 10% (v/v). Simultaneously the compounds I to XVII (see Table 1 A to 1D and 2) are added in a concentration range of 0 to 20 µM. Triplicates are measured for each concentration. Cells and compounds (see Table 1 A to 1D, and 2) are incubated for a period of 48 hours in a volume of 140 µl cell culture medium (10% (v/v) FCS, 2 mM Glutamin, DMEM). The compounds are solved in 60% to 100% (v/v) DMSO at a concentration of 2 mM. The final DMSO concentration under assay conditions never exceeded 1% (v/v). Throughout the assay cells are kept in a humidified incubator (95% rH) at 37°C and 5% CO₂. The final cell number is determined after 48 hour of incubation. Therefore 140 µl CellTiter-Glo^{®}Reagent is added in each well. The contents are mixed on an orbital shaker for 2 minutes at room temperature. After subsequent incubation for additional 10 minutes at room temperature the luminescence signal was recorded within 30 min. EC50 values are calculated using the four-parametric Hill equation (Sigma Plot).

Control values are: (1) 10% (v/v) FCS, 2 mM Glutamin, DMEM -> maximum cell number and (2) 2 mM Glutamin, DMEM -> initial cell number.

E-3-{4-[4-(3,4-ethylendioxphenyl)-thiazol-2-yl]-piperidin-1-yl}-1-(3,4-methylendioxyphenyl)-3-oxo-prop-1-ene (compound I in Table 1A) blocks the proliferation of HuH7 with an EC50 of 0.33 µM. Furthermore, HepG2 and Hep3B are inhibited with an EC50 of 0.9 and 0.3 µM, respectively.

**Table 1A: Inhibition of HuH7 proliferation by compounds I to IV (EC50: effective concentration reducing cell proliferation by 50%)**

| Compound No. | EC50/µM | Chemical Structure |
|---|---|---|
| I | 0.33 | 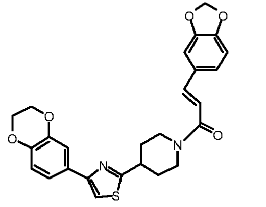 |
| II | 12.37 | 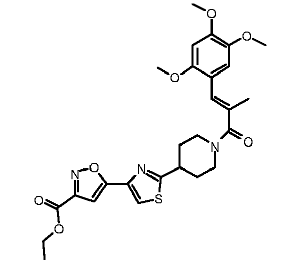 |
| III | 1.61 | 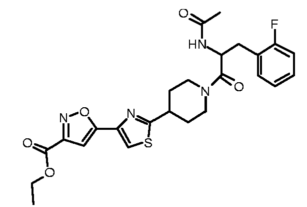 |
| IV | 5.02 | 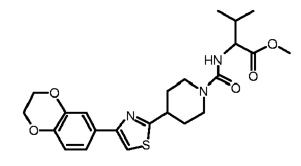 |

**Table 1B: Inhibition of HuH7 proliferation by compounds V to IX (EC50: effective concentration reducing cell proliferation by 50%).**

| Compound No. | EC50/µM | Chemical Structure |
|---|---|---|
| V | 10.7 | 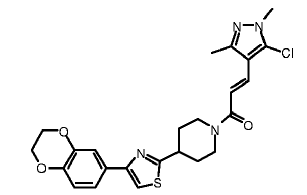 |
| VI | 20.7 | 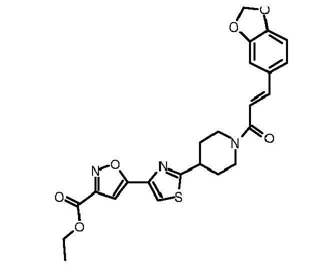 |
| VII | 6.2 | 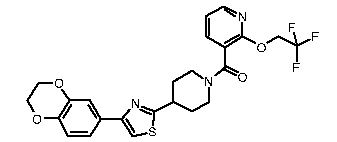 |
| VIII | 1.89 | 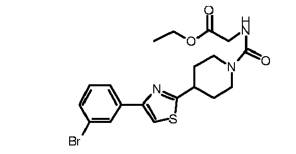 |
| IX | 3.93 | 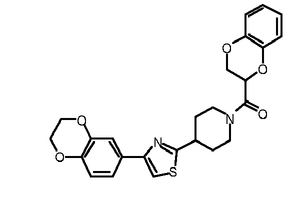 |

**Table 1C: Inhibition of HuH7 proliferation by compounds X to XV (EC50: effective concentration reducing cell proliferation by 50%).**

| Compound No | EC50/µM | Chemical Structure |
|---|---|---|
| X | 1.93 | 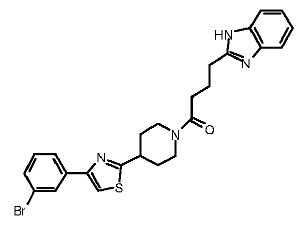 |
| XI | 3.35 | 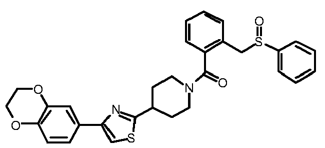 |
| XII | 5.76 | 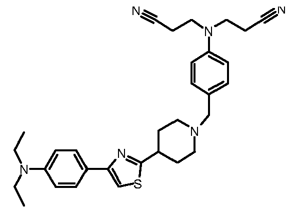 |
| XIII | 6 | 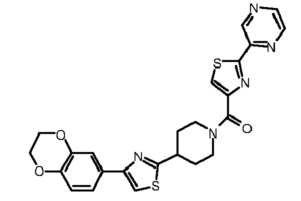 |
| XIV | 29 | 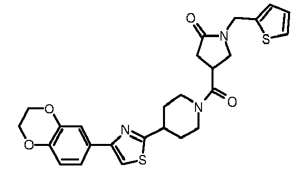 |
| XV | 4.94 | 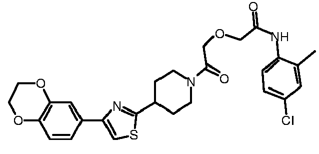 |

**Table 1D: Inhibition of HuH7 proliferation by compounds XVI to XV (EC50: effective concentration reducing cell proliferation by 50%).**

| Compound No. | EC50/µM | Chemical Structure |
|---|---|---|
| XVI | 24.9 | 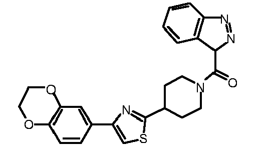 |
| XVII | 0.63 | 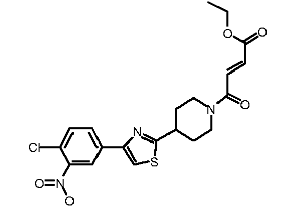 |

Furthermore, compounds of the formula 1 also show inhibition of cell proliferation of other tumour cell lines (see Table 2).

**Table 2: Inhibition of DAN-G, SK-Br-3, HeLa, and CX-2 proliferation by compounds I and V (EC50: effective concentration reducing cell proliferation by 50%; >>: much greater than).**

| Cell line | DAN-G | CX-2 | HeLa | Sk-Br-3 |
|---|---|---|---|---|
| Compound No. | EC50/µM | EC50/µM | EC50/µM | EC50/µM |
| I | 4.69 | 19.1 | 14.8 | 10.8 |
| V | 11.05 | >>20 | 18.7 | >>20 |

On the basis of these studies a compound of formula (1) according to the invention shows therapeutic efficacy especially against cancer, preferably in liver and pancreas.

## Claims

1. A compound of formula (1), 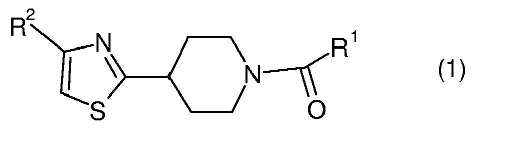 wherein
R¹ represents optionally substituted amino, optionally substituted phenyl, optionally substituted heteroaryl, optionally substituted heterocyclyl, optionally substituted phenyl- or heteroaryl-alkyl, optionally substituted phenylaminocarbonyl-alkoxyalkyl or a residue of formula (2), 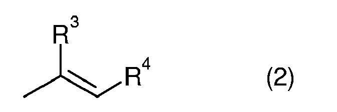 wherein R³ is hydrogen or alkyl and R⁴ is optionally substituted phenyl, optionally substituted heteroaryl, carboxy, alkoxycarbonyl, optionally substituted aminocarbonyl or cyano,
and R² represents optionally substituted phenyl or optionally substituted heteroaryl;
and salts thereof.

2. The compound of formula (1) according to claim 1 wherein R¹ is phenyl-C₁-C₄-alkyl with a halo substituent on phenyl and an amino substituent on alkyl, or benzimidazolyl-C₁-C₄-alkyl.

3. The compound of formula (1) according to claim 1 wherein R¹ is a residue of formula (2), 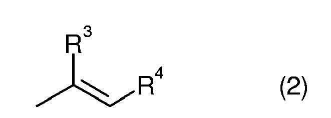 wherein R³ is hydrogen or C₁-C₄-alkyl and R⁴ is optionally substituted phenyl, optionally substituted pyrazolyl, or C₁-C₄-alkoxycarbonyl.

4. The compound of formula (1) according to claim 1 wherein R² represents phenyl substituted by halo, nitro, dialkylamino or ethylenedioxy.

5. The compound of formula (1) according to claim 1 wherein
R¹ is C₁-C₄-alkylcarbonyl-C₁-C₇-alkyl, phenyl substituted by di(cyano-C₁-C₄-alkyl)amino, phenylsulfoxy-C₁-C₄-alkyl, fluoro-C₁-C₄-alkoxy-pyridyl, pyrazinyl-thiazolyl, or 3-benzopyrazolyl, oxo-N-(heteroaryl-alkyl)-pyrrolidinyl, benzo-1,4-dioxolanyl, fluorophenyl-acetylamino-C₁-C₄-alkyl, halo- and C₁-C₄-alkyl substituted phenylaminocarbonyl-C₁-C₄-alkoxy-C₁-C₄-alkyl, a residue of formula (2) and R³ is hydrogen or methyl and R⁴ is trimethoxyphenyl, methylenedioxyphenyl, pyrazolyl substituted by methyl and halo, or C₁-C₄-alkoxycarbonyl; and
R² is 4-chloro-3-nitrophenyl, 3-bromophenyl, 4-diethylaminophenyl, 3,4-ethylenedioxyphenyl, or 3-ethoxycarbonyl-5-isoxazolyl.

6. Compounds of formula (1) according to claim 1 selected from the group consisting of
the compound of formula (1) wherein R¹ is a residue of formula (2), R² is 3,4-ethylenedioxyphenyl, R³ is hydrogen and R⁴ is 3,4-methylenedioxyphenyl;
the compound of formula (1) wherein R¹ is a residue of formula (2), R² is 4-chloro-3-nitrophenyl, R³ is hydrogen and R⁴ is ethoxycarbonyl;
the compound of formula (1) wherein R¹ is 3-(1 H-benzimidazol-2-yl)-propyl and R² is 3-bromo-phenyl;
and
the compound of formula (1) wherein R¹ is 2-(2-fluorophenyl)-1-(acetylamino)-methyl and R² is 3-ethoxycarbonyl-isoxazol-5-yl.

7. A pharmaceutical preparation comprising a compound of anyone of claims 1 to 6.

8. A compound according to anyone of claims 1 to 6, a prodrug or a pharmaceutically acceptable salt thereof for use in the treatment of hyperproliferative diseases.

9. Use of a compound of formula (1), a prodrug or a pharmaceutically acceptable salt of such a compound for the preparation of a pharmaceutical composition for the treatment of a hyperproliferative disease.

10. A method for the treatment of a hyperproliferative disease, which comprises administering a compound of formula (1), a prodrug or a pharmaceutically acceptable salt thereof according to anyone of claims 1 to 6 in a quantity effective against said disease, to a warm-blooded animal requiring such treatment.
